# EUROPEAN PATENT APPLICATION

(11) **EP 3 915 678 A1**
(43) Date of publication of application: **01.12.2021**
(21) Application number: 20177444.5
(22) Date of filing: 29.05.2020
(51) Int. Cl.: B01J 29/06, B01J 29/08, B01J 29/10, B01J 29/12, B01J 29/14, B01J 29/70, B01J 29/72, B01J 29/74, B01J 29/76, B01J 37/00, B01J 37/18, C07C 1/20, C07C 29/153, C07C 41/09, C10G 2/00, C10K 3/02

(54) **SORPTION-ENHANCED REVERSE WATER-GAS SHIFT CATALYST**

(71) Applicant: Nederlandse Organisatie voor toegepast- natuurwetenschappelijk Onderzoek TNO, 2595 DA Den Haag (NL)
(72) Inventor: The designation of the inventor has not yet been filed
(74) Representative: Nederlandsch Octrooibureau

(57) **Abstract**

The present invention concerns a multifunctional catalyst for the conversion of CO₂ into useful products, such as CO via the reverse water gas shift reaction. The catalyst according to the invention efficiently combined a water sorption functionality with at least one a catalytic functionality into a single particle, by having a solid water sorbent impregnated with at least one metal capable of converting CO₂ from a gaseous mixture comprising H₂ and CO₂. The catalyst according to the invention allows for higher selectivity in the conversion of CO₂, at more lenient conditions in terms of temperature and pressure, and improved stability of the catalyst itself. The invention also concerns a process for converting CO₂, utilizing the catalyst and the use of the catalyst in the conversion of CO₂.

## Description

### TECHNICAL FIELD

The present invention is in the field of gaseous processes and relates to a multifunctional catalyst for the conversion of CO₂ in useful products, such as via the reverse water gas shift reaction (RWGS). The invention further concerns the use of the catalyst in the conversion of CO₂ reaction and corresponding processes.

### BACKGROUND OF THE INVENTION

Global energy consumption has soared due to increasing population and industrialization, and anthropogenic CO₂ emissions have rapidly grown because the main energy resources being consumed today are fossil fuels. Increasing CO₂ concentration in the atmosphere is leading to global warming and a range of environmental problems. International efforts have been proposed to reduce CO₂ emissions, such as the multinational Paris agreement. At the same time, many studies have focused on the development and application of renewable energy sources and on technologies for capturing and utilizing CO₂.

The effective utilization of CO₂ is hampered by its low reactivity (low equilibrium conversion) in the production of valuable building blocks for larger materials. For example, the conversion of CO₂ to dimethyl ether or methanol at CO₂:H₂ 1:3 suffers from a single-pass conversion of only about 8 % (275 °C, 40 bar), while the synthesis from a CO enriched feed significantly improves conversion, to a single-pass conversion of more than 50% at the same conditions. The utilization of CO₂ would thus be greatly facilitated by an efficient conversion into CO. For example, the reverse water-gas shift (RWGS) reaction is the most prominent manner for converting CO₂ into CO, at the expense of H₂: H₂ + CO₂ ↔ CO + H₂O (ΔH₀41kJ / mol). The RWGS reaction produces CO, which is a very flexible chemical intermediate. For instance, the reaction can be combined with Fischer-Tropsch technology to produce liquid hydrocarbon fuels. In addition, the reaction can also be used in the production of methane, methanol, and dimethyl ether, which have potential as new generation transport fuels, and in the production of valuable chemicals such as paraffins, olefins, and aromatics.

The RWGS is an endothermic reaction. The forward reaction is beneficiated and the chemical balance is shifted in the direction of the products (i.e. CO + H₂O) only at high temperatures. Temperatures above 800 °C are generally required to achieve conversions of 50%. The selectivity towards CO at lower temperatures (300 °C and 1 bar(a)) can be improved by using sorption-enhanced technology, see Haije, W. et al. in "Efficient production of solar fuel using existing large scale production technologies" (2011, Environ. Sci. Technol.). Carvill, B. T. et al. in "Sorption-enhanced reaction process" (1996, AlChE Journal) describe the use of a fixed packed column of an admixture of a catalyst and a sorbent to enable CO₂ conversion at an operating temperature of 250 °C and a pressure 3.8 bar. Whereas H₂O is the main by-product of many equilibrium limited reactions, such as the water-gas shift reaction and dimethyl ether synthesis, removal thereof *in situ* will result in significant conversion enhancements and thus process intensification. This is based on Le Chatelier's principle, according to which the reactant conversion to products in an equilibrium limited reaction is increased by selectively removing reaction products. Yang, X. et al. in "Promotion effects of potassium (K) on the activity and selectivity of Pt/LTL catalysts for reverse water gas shift reaction" (2017, Applied Catalysis B: Environmental) describe that controlled addition of potassium promoter to zeolite L-supported platinum catalyst was beneficial for the catalytic activity and selectivity in the reduction of CO₂. The vicinity of K and Pt enhanced the CO₂ adsorption and reaction at the reaction site. However, this promotion effect is not based on Le Chatelier's principle and therefore not a sorption-enhanced reaction process. Also, the conversion of CO₂ in the work by Yang et al was at low temperatures, i.e. 250 - 335 °C, still largely insignificant (<5 %).

The present invention provides in the need to further improve the conversion of CO₂ in valuable products, such as via the RWGS reaction.

### SUMMARY OF THE INVENTION

The inventors have developed a multifunctional catalyst that outperforms conventional catalysts in the conversion of CO₂. The catalyst according to the invention allows for higher conversion to product formation under more lenient reaction conditions in terms of temperature, which avoids temperature-induced catalyst deactivation, and at pressures that in general align much better with the reaction conditions required for the synthesis of further products in subsequent processes. Moreover, the catalyst according to the invention allows for higher selectivity towards CO formation at the high pressures required to react the CO to other products in sequential process steps. The multifunctional catalyst according to the invention comprises a solid water sorbent impregnated with at least one metal capable of catalysing the conversion of CO₂ from a gaseous mixture comprising H₂ and CO₂ into a useful product. The metal content in the impregnated molecular sieve is not more than 40% based on total weight of the catalyst. The sorbent is a molecular sieve having a Si/AI molar ratio below 50 and containing pores having a diameter in the range of 3 to 20 Å.

The catalyst according to the invention can be used in the formation of several valuable products from a gaseous mixture comprising CO₂ and H₂, such as CO, lower alcohols (e.g. methanol, ethanol), ethers (e.g. dimethyl ether, diethyl ether) and hydrocarbons. The multifunctional catalyst of the invention allows for higher selectivity in the conversion of CO₂, at more lenient conditions in terms of temperature and pressure. In addition, this technology overcomes problems typically encountered when using catalyst systems based on a mixture of components having very different physical-chemical properties (e.g. separation, uniformity, upscaling, attrition, stability), which are avoided with the homogenous catalyst according to the invention.

The invention provides a versatile approach towards the formation of any of these possible products. Careful selection of the elements (e.g. sorbent, such as pore size and Si/AI ratio; type of metal catalyst) of the multifunctional catalyst according to the invention, as well as the composition of the feedstock (e.g. CO₂ to H₂ ratio, presence and content of CO) provides optimal conversion into the desired product. Also, the invention provides the joining of multiple (in particular three) functionalities, wherein at least two functionalities are provided by the catalytic particle of the invention and the third functionality is provided either by a separate catalytic particle, or is combined within the same particle.

More specifically, the invention can be defined according to the following preferred embodiments:
1. Use of a multifunctional catalyst, comprising a solid water sorbent impregnated with at least one metal capable of converting CO₂ from a gaseous mixture comprising H₂ and CO₂, as catalyst for the conversion of CO₂, wherein the sorbent is a molecular sieve having a Si/AI molar ratio below 50 and containing pores having a diameter in the range of 3 to 20 Å and the metal content in the catalyst is not more than 40% based on of total weight of the multifunctional catalyst.
2. The use according to embodiment 1, wherein the conversion of CO₂ involves a reverse water gas shift reaction (RWGS) to form CO, a hydrogenation reaction to form methanol or ethanol, a Fisher-Tropsch reaction to form alkanes, a Fisher-Tropsch reaction to form alkenes, a Methanol to Olefins (MTO) reaction to form alkenes, an aromatization reaction to form aromatic hydrocarbons or a hydrogenation reaction to form methane, optionally wherein the hydrogenation reaction is accompanied with a dehydration reaction to convert methanol into dimethyl ether or ethanol into diethyl ether.
3. A process for the conversion of CO₂ from a gaseous feedstock comprising H₂ and CO₂, comprising the step of subjecting the gaseous feedstock to a multifunctional catalyst, comprising a solid water sorbent impregnated with at least one metal capable of converting CO₂ from a gaseous mixture comprising H₂ and CO₂, wherein the sorbent is a molecular sieve having a Si/AI molar ratio below 50 and containing pores having a diameter in the range of 3 to 20 Å and the metal content in the catalyst is not more than 40% based on total weight of the multifunctional catalyst.
4. The process according to embodiment 3, wherein the conversion of CO₂ involves a reverse water gas shift reaction (RWGS) to form CO, a hydrogenation reaction to form methanol or ethanol, or a Fisher-Tropsch reaction to form alkanes, a Fisher-Tropsch reaction to form alkenes, a Methanol to Olefins (MTO) reaction to form alkenes, an aromatization reaction to form aromatic hydrocarbons or a hydrogenation reaction to form methane, optionally wherein the hydrogenation reaction is accompanied with a dehydration reaction to convert methanol into dimethyl ether or ethanol into diethyl ether.
5. The process according to embodiment 4, wherein the conversion is a RWGS reaction.
6. The process according to any one of embodiments 3 - 5, wherein the process takes place at a temperature in the range of 100 - 500 °C, preferably in the range of 200 - 400 °C.
7. The process according to any one of embodiments 3 - 6, wherein the reaction takes place at a pressure in the range of 0.5 - 20 MPa, preferably 1 - 10 MPa, more preferably 1.5 - 5 MPa.
8. The process according to any one of embodiments 3 - 7, wherein the molar ratio of H₂ to CO₂ in the feedstock is in the range of 10:1 - 1:5, preferably 5:1 - 1:1, more preferably about 4:1, and/or wherein the feedstock further comprises CO.
9. The process according to any one of embodiments 3 - 8, further comprising a regeneration step of the catalyst wherein the pressure is reduced by at least 0.1 x 10⁶ Pa, preferably to a pressure in the range of 0.1 x 10⁶ Pa -0.3 x 10⁶ Pa and/or the temperature is increased by at least 20 °C, preferably to a temperature in the range of 370 °C - 500 °C.
10. A multifunctional catalyst, comprising a solid water sorbent impregnated with at least one metal capable of catalyzing the conversion of CO₂ from a gaseous mixture comprising H₂ and CO₂, wherein the sorbent is a molecular sieve having a Si/AI molar ratio below 50 and containing pores having a diameter in the range of 3 to 20 Å and the metal content in the catalyst is not more than 40% based on total weight of the multifunctional catalyst.
11. The use according to embodiment 1 or 2, the process according to any one of embodiments 3 - 9, or the catalyst according to embodiment 10, wherein the sorbent has a Si/AI molar ratio in the range of 1 and 2.0, more preferably in the range of 1 and 1.5, most preferably the Si/AI molar ratio is 1, preferably wherein the sorbent is a zeolite selected from LTA zeolite, X-type zeolite and mixtures thereof, preferably the multifunctional component comprises LTA-4A zeolite and/or zeolite 13X.
12. The use according to embodiment 1, 2 or 11, the process according to any one of embodiments 3 - 9 and 11, or the catalyst according to embodiment 10 or 11, wherein the pores have a diameter in the range of 4 to 8 Å.
13. The use according to embodiment 1, 2, 11 or 12, the process according to any one of embodiments 3 - 9, 11 and 12, or the catalyst according to any one of embodiments 10- 12, wherein the internal and external surfaces of the sorbent are impregnated with the at least one metal catalyst, and/or wherein the sorbent is impregnated with 0.1 - 40 wt%, preferably 1 - 30 wt%, of the metal, based on total weight of the multifunctional catalyst.
14. The use according to any one of embodiments 1, 2, 11 - 13, the process according to any one of embodiments 3 - 9, 11 - 13, or the catalyst according to any one of embodiments 10 - 13, wherein the at least one metal is selected from the group consisting of Pt, Cs, Cu, Rh, Au, Zn, Co, Fe, Mo, Zn, Mn, Ru, Ir, Os, Ag, Al, Cr, Li, Na, K, Rb, Cs, Ce, Re, In, Zr, Ni, Mg, Al, Pd, Ga and mixtures thereof, preferably from Pt, Cu, Rh, Ir, Ru, Pd, Os, Cs, Fe, Zn and mixtures thereof, more preferably from the group consisting of Pt, Zn, Fe, Cs, Cu and mixtures thereof.
15. The use according to any one of embodiments 1, 2, 11 - 14, the process according to any one of embodiments 3 - 9, 11 - 14, or the catalyst according to any one of embodiments 10 - 14, wherein the solid sorbent is further impregnated with a second metal capable of catalyzing the synthesis of dimethyl ether from methanol or diethyl ether from ethanol.

### DETAILED DESCRIPTION

Accordingly, a first aspect of the present invention relates to a multifunctional catalyst for the conversion of CO₂. The catalyst comprises a solid water sorbent impregnated with at least one metal capable of catalysing the conversion of CO₂ from a gaseous mixture comprising H₂ and CO₂. The content of metal catalyst in the impregnated solid water sorbent is not more than 40% based on weight of the catalyst. The sorbent is a molecular sieve having a Si/AI molar ratio below 50 and contains pores (or cages) having a diameter in the range of 3 to 20 Å.

In a second aspect, the present invention concerns the use of the catalyst according to the invention as catalyst for the conversion of CO₂. In a third aspect, the present invention concerns a process for the conversion of CO₂ from a gaseous feedstock comprising H₂ and CO₂, comprising contacting the gaseous feedstock with the catalyst according to the invention.

Whenever reference is made to the catalyst according to the invention, or preferred embodiments thereof, this equally applies to the use and processes according to the invention. Also, whenever reference is made to the use according to the invention, this equally applies to the processes according to the invention.

### The catalyst

First and foremost, the present invention concerns a multifunctional catalyst for the conversion of CO₂. The catalyst according to the invention is a multifunctional catalyst, comprising a solid water sorbent impregnated with at least one metal capable of converting CO₂, wherein the sorbent is a molecular sieve-having a Si/AI molar ratio below 50 and containing pores having a diameter in the range of 3 to 20 Å and the metal content in the catalyst is not more than 40% based on total weight of the multifunctional catalyst. The multifunctional nature of the catalyst resides in its sorption capacity of water and its catalytic capacity of converting CO₂ and optionally the further conversion of the intermediate products from CO₂.

The multifunctional catalyst according to the invention gives high selectivities towards the products of the conversion of CO₂, even at high pressures of 15 - 200 bar and relatively low temperatures such as 225 - 335 °C. For example, the conversion of CO₂ into CO via the RWGS reaction ran smoothly at 300 °C at high pressures of 15 - 25 bar. Furthermore, the multifunctional catalyst according to the invention is homogenous, in that it contains one type of particles, while conventional multifunctional catalyst systems contain one type of particle with sorbent and other type(s) of particle(s) with catalyst. Such a heterogeneous particulate gives problems with uniformity, separation, scalability and instability of the system. Such problems are primarily caused by differences in hardness between the hard catalyst particles and soft sorbent particles as well as mass transfer limitations. For example, when such a convention system of sorbent particles and catalyst particles are mixed together in a fluidized bed reactor, the sorbent particles will quickly pulverize into a powder and lose their sorption capacity. This is avoided with the multifunctional catalyst according to the invention, which contains one type of particles, sorbent impregnated with metal catalyst.

The inventors have achieved these effects by using a multifunctional reactive sorbent, which is a material that both catalyses the conversion of CO₂ and adsorbs water. The multifunctional reactive sorbent comprises a specific water sorbent and a metal impregnated on the surface of the sorbent, preferably in the pores thereof. The zeolite thus contains both reactive pockets wherein CO₂ is converted, as well as areas wherein H₂O can be adsorbed. Since the sorbent and catalyst are thus integrated into one material, the conversion of CO₂ can easily be scaled up.

The multifunctional catalyst is a solid material, preferably a granulate. The granules may be spherical, structured (e.g. composites, tandem, hybrid), pellets and the like. Advantageously, the granulate contains particles having a diameter of 150 - 600 µm, preferably in the range of 200 - 450 µm. The skilled person understands how to size the particulate based on internal and external mass transfer criteria. Thus, in a preferred embodiment, the catalyst according to the invention is a particle comprising a sorbent impregnated with at least one metal capable of catalyzing the conversion of CO₂.

The sorbent part of the catalysts is a solid water sorbent. The sorbent is a molecular sieve. Even though any molecular sieve capable of sorbing water molecules is suitable in the context of the present invention, preferably a zeolite (microporous or mesoporous or mixtures thereof) is used as sorbent. Zeolites having a pore diameter in the range of 3 to 20 Å are known to the skilled person for the selective sorption of water molecules. Preferably, the pores have a diameter in the range of 4 to 9 Å. The pore structure of the zeolites may also contain cages, which could be beneficial in steering the product selectivity towards the desired product.

In a preferred embodiment, the solid water sorbent is a zeolite selected from LTA zeolite, FAU X-type, FAU Y-type including ultrastable Y (USY), EMT, BEA, CHA (ALPO, APSO, SAPO, SSZ-13) zeolite, as well as and mixtures and derivatives (dealuminated, alkaline and acid treated, promoted and stabilized with rare earth metals, hybrids, partially exchanged etc.), and natural counterparts thereof. Zeolite LTA (Linde Type A) belongs to the family of aluminosilicate molecular sieves. It is characterized by the formula |Na⁺₁₂(H₂O)₂₇|₈[Al₁₂Si₁₂O₄₈]₈ (International Zeolite Association-IZA). Sodium ions in zeolite A can be exchanged with other cations such as lithium (Li-LTA), potassium (K-LTA), or calcium (Ca-LTA). When one ion is exchanged by the other, the size of the pore opening changes. Exchanging the potassium ions in zeolite LTA-3A with the smaller sodium ions results in zeolite LTA-4A having an increased diameter of the pores, which determines its accessibility for molecular permeation. When sodium is exchanged with Ca²⁺ in a 2:1 fashion, zeolite 5A is obtained.

Zeolite X belongs to the family of aluminosilicate molecular sieves with a faujasite-type structure (FAU). It is characterized by the formula |(Ca²⁺,Mg²⁺Na⁺₂)₂₉(H₂O)₂₄₀|[Al₅₈Si₁₃₄O₃₈₄] - FAU (International zeolite association -IZA). Structurally, LTA zeolites and X-type are relatively similar. Both consist of β-cages, containing square and hexagon faces. In both types these cages are linked to create very hydrophilic supercages. The inventors obtained optimal results with LTA-4A zeolite and zeolite 13X.

The molecular sieve used as sorbent in the catalyst of the invention has a Si/AI molar ratio below 50, such as in the range 1 - 50. The preferred Si/AI molar ratio may depend on the specific product of the CO₂ conversion reaction, as further specified below. Generally speaking, it is preferred that the Si/AI ratio is below 10, preferably below 5, more preferably in the range of 1 - 4.0, most preferably the Si/AI ratio is in the range of 1 - 2. In an alternative preferred embodiment, the Si/AI ratio is in the range of 10 - 50, more preferably in the range of 20 - 30. With higher Si/AI molar ratios, the hydrophilicity and water sorption capacity of the sorbent is reduced and the multifunctionality of the catalyst of the invention is impaired. Zeolites having a Si/AI ratio above 2 or even 10 are suitable. Their reduced Al content leads to a slightly lower water sorbent capacity, with increases the acidic nature of the zeolite, which is beneficial for (alcohol) dehydration to e.g. dimethyl ether or diethyl ether. Hence, especially for the trifunctional catalysts defined further below, the Si/AI ratio may be above 2.5, such as in the range of 2.5 - 50, preferably in the range 5 - 30. If needed, the Si/AI ratio could be increased by leaching of Al from the zeolite (dealumination). Such a procedure is known to the skilled person.

The sorbent is impregnated with at least one metal capable of catalysing the conversion of CO₂ from a gaseous mixture comprising H₂ and CO₂. In the context of the present invention, it is not required that the entire surface of the sorbent is impregnated. Preferably, the sorbent is impregnated with 0.1 - 40 wt%, more preferably 1 - 30 wt% of metal, based on total weight of the catalyst. The optimal metal content may vary depending on the desired product and the specific metal or combination of metals used. In a preferred embodiment, at least the internal surface (the pores) are impregnated, more preferably both the internal surface (within the pores) and the external surface (the outside of the particles) are impregnated with the metal.

Several reactions are known to convert CO₂ into useful products. Such useful products may be selected from such as CO, lower alcohols (e.g. methanol, ethanol), lower ethers (e.g. dimethyl ether, diethyl ether), alkanes. In a preferred embodiment, the conversion of CO₂ involves the reverse water gas shift (RWGS) reaction, most preferably the conversion of CO₂ is the RWGS reaction and the product is CO. A further suitable reaction is the hydrogenation of CO₂ into alcohols, preferably methanol or ethanol, most preferably methanol. A further suitable conversion of CO₂ involves the hydrogenation of CO₂ into alcohols and the subsequent dehydration into ethers, preferably dimethyl ether or diethyl ether, most preferably dimethyl ether. A further suitable conversion of CO₂ involves Fischer-Tropsch reaction to form hydrocarbons or alkanes.

The skilled person is capable of selecting a metal, or combination of metals, that is capable of catalysing the desired conversion of CO₂. In a preferred embodiment, the sorbent is impregnated with at least one metal selected from the group consisting of Pt, Cs, Cu, Rh, Au, Zn, Co, Fe, Mo, Zn, Mn, Ru, Ir, Os, Ag, Al, Cr, Li, Na, K, Rb, Cs, Ce, Re, In, Zr, Ni, Mg, Al, Pd, Ga and mixtures thereof, preferably from the group consisting of Pt, Cu, Rh, Ir, Ru, Pd, Os, Cs, Fe and Zn. More preferably, the metal is selected from the group consisting of Pt, Cs, Cu, Fe, Zn, Rh and mixtures thereof. Even more preferably, the metal is selected from the group consisting of Pt, Cs, Cu, Fe and mixtures thereof. Most preferably, the metal is Pt or a mixture of Pt, Cs and Cu or Fe.

In one especially preferred embodiment, the sorbent is impregnated with Pt. When Pt is present, the molecular sieve is preferably impregnated with 0.1 - 8 wt%, preferably 0.2 - 3 wt%, more preferably 0.5 - 2 wt% Pt, based on the total weight of the multifunctional catalyst. When Pt is present, the metal that is impregnated on the sorbent may contain one or more further metals, preferably selected from the group consisting of Rh, Ru, Ir, Pd, Os, Fe, Cs, Cu, Zn, or other transition metals.

In another especially preferred embodiment, the sorbent is impregnated with Cu, preferably a combination of Cu with Cs and/or Zn. When Cu is present, the molecular sieve is preferably impregnated with 1 - 40 wt%, preferably 2 - 30 wt%, more preferably 5 - 25 wt% Cu, based on the total weight of the multifunctional catalyst. The inventors have found that the impregnation of the solid water sorbent with a mixture of Cs and Cu or Pt is particularly advantageous for RWGS. Although the inventors do not wish to be bound by theory, it is believed that the presence of Cs blocks the side reactions that may lead to the formation of methanol or dimethyl ether, as such increasing the selectivity of the RWGS reaction to convert CO₂ in CO. In case both Cu and Cs are present, it is preferred that the multifunctional catalyst comprises a molecular sieve impregnated with Cu and Cs in a molecular ratio of 47:1 to 1:1, preferably 6:1 to 2:1. In case both Cu and Zn are present, it is preferred that the multifunctional catalyst comprises a molecular sieve impregnated with Cu and Zn in a molecular ratio of 4:1 to 1:2, preferably 2:1 to 2:3.

The inventors have found that the formation of the desired product can be optimized by selecting the optimal combination of a metal (or combination of metals) and sorbent. In other words, the catalyst particle can be tuned to the formation of the desired product. Examples of such optimal combinations are provided below. As such, the present invention provides a versatile approach towards the conversion of CO₂, wherein the metal (or combination of metals) and the sorbent may be selected to provide optimal conversion of CO₂ into the desired product.

In an especially preferred embodiment, the conversion of CO₂ involves the RWGS reaction. In the context of this embodiment, the metal is capable of catalysing the RWGS reaction. Preferably, the metal is selected from the group consisting of Pt, Cs, Cu, Rh, Au, Zn, Co, Fe, Mo and mixtures thereof, more preferably, the metal is selected from the group consisting of Pt, Cs, Cu, Fe and mixtures thereof. Most preferably, the metal is Pt, Pt or a mixture of Cs and Cu, or a mixture of Pt, Cs and Cu or Fe. In another preferred embodiment, the metal comprises at least Cs, which was found to be suitable to suppress hydrogenation of CO to produce methanol and as such increase the yield of CO. Preferably, the Cs content is in the range of 0.1 - 2.5 wt%, preferably 1 - 2 wt%, based on total weight of the catalyst.

In an especially preferred embodiment, the metal is Pt. This metal is optimally combined with a zeolites having a pore diameter in the range of 3 to 5 Å, preferably of about 4 Å, as sorbent. In another especially preferred embodiment, the metal is a mixture of Cu and Cs. This metal is optimally combined with a zeolites having a pore diameter in the range of 3 to 10 Å, preferably of about 4 Å, as sorbent. The Si/AI ratio for performing a RWGS reaction is preferably below 2.5, more preferably in the range of 1 - 2. Preferably, the sorbent in the multifunctional catalyst for the RWGS reaction is an LT-4Å zeolite or 13X zeolite, most preferably an LT-4Å zeolite. The inventors have obtained excellent results in catalysing the RWGS reaction with zeolite LTA-4A or 13X, impregnated with 1 - 4 wt% Pt, based on total weight of the catalyst.

In an alternative preferred embodiment, the conversion of CO₂ involves hydrogenation to form methanol. In the context of this embodiment, the metal is capable of catalysing the hydrogenation of CO₂. Preferably, the metal is selected from the group consisting of Cu, Au and mixtures thereof, more preferably, the metal is Au. Preferably, the metal in the context of this embodiment does not comprise Cs. The metal is optimally combined with a zeolites having a pore diameter in the range of 5 - 10 Å, preferably in the range of 7 - 9 Å, as sorbent. Most preferably, the sorbent in the multifunctional catalyst for the RWGS reaction is a zeolite having a Si/AI ratio in the range of 1 - 2, more preferably in the range 1 - 1.5. The sorbent is preferably selected from the group consisting of faujasite, zeolite 13X, zeolite Y, BEA, EMT zeolites.

In an alternative preferred embodiment, the conversion of CO₂ involves hydrogenation to form ethanol. Herein, two molecules of methanol or one molecule of methanol and one molecule of CO couple to form a carbon-carbon bond. Zeolites with high acid site strength are preferred, such as dealuminated zeolites with Si/AI > 2.5. In the context of this embodiment, the metal is capable of converting CO₂, optionally together with CO, into ethanol. Preferably, the metal is selected from the group consisting of Cu, Zn, Mn, Rh, Ru, Pt, Ir, Os, Au, Ag, Al, Co, Cr, Fe, alkali metals and mixtures thereof, more preferably, the metal is selected from the group consisting of Cu, Zn, Cs, Li, K, Al, Fe, Rh and mixtures thereof. In a preferred embodiment, the metal is a mixture of (i) Cu and/or Zn, (ii) one or more of Cs, Li, K and Al and (iii) Fe and/or Rh. This metal is optimally combined with a zeolites having a pore diameter in the range of 5 - 10 Å, preferably in the range of 7 - 9 Å, as sorbent. Most preferably, the sorbent in the multifunctional catalyst is a zeolite having a Si/AI ratio in the range of 3 - 5. The sorbent is preferably selected from the group consisting of zeolite BEA and FAU (ultrastabilized Y (USY), dealuminated X).

In an alternative preferred embodiment, the conversion of CO₂, optionally together with CO, involves hydrogenation to form alkanes via Fischer-Tropsch synthesis. In the context of this embodiment, the metal is capable of converting CO₂ into hydrocarbons. Preferably, the metal is selected from the group consisting of Co, Fe, Mo, Ru and mixtures thereof, and may further comprise K, Na, Li, Cs, Mn and mixtures thereof.. These metals are optimally combined with a zeolite having a pore diameter in the range of 5 - 10 Å, preferably in the range of 7 - 9 Å, as sorbent. Most preferably, the sorbent in the multifunctional catalyst for Fischer-Tropsch synthesis is a zeolite having a Si/AI ratio above 5. The sorbent is preferably selected from the group consisting of zeolite BEA, FAU (Y including ultrastabilized Y (USY), 13X (including dealuminated 13X)).

In an alternative preferred embodiment, the conversion of CO₂, optionally together with CO, involves hydrogenation to form alkenes via Fischer-Tropsch synthesis. In the context of this embodiment, the metal is capable of converting CO₂ into hydrocarbons. Preferably, the metal is selected from the group consisting of Co, Fe, Mo, Ru and mixtures thereof, and may further comprise K, Ce, Na, Li, Cs, Mn and mixtures thereof to promote the formation of olefins. The skilled person is capable to fine-tune the amount of the metals towards the formation of alkenes over alkanes. This metal is optimally combined with a zeolite having a pore diameter in the range of 5 - 13 Å, preferably in the range of 7 - 9 Å, as sorbent. Most preferably, the sorbent in the multifunctional catalyst is a zeolite having a Si/AI ratio above 5. The sorbent is preferably selected from the group consisting of zeolite CHA, BEA, FAU (Y including ultrastabilized Y (USY), 13X (including dealuminated 13X)).

In an alternative preferred embodiment, the conversion of CO₂, optionally together with CO, involves hydrogenation to form alkenes via Methanol to Olefins (MTO) synthesis. In the context of this embodiment, the metal is capable of converting CO₂ into C₂-C₄ olefins. Preferably the metal is selected from the group consisting of Cu, Re, In, Zr, Au, Co, Fe, Mo, Ru and mixtures thereof, and may further comprise K, Ce, Na, Li, Cs, Mn and mixtures thereof to promote the formation of olefins. These metals are optimally combined with a zeolite having a pore diameter in the range of 4 - 13 Å, preferably in the range of 4 - 9 Å, as sorbent. Most preferably, the sorbent in the multifunctional catalyst is a zeolite having a Si/AI ratio above 5. The sorbent is preferably selected from the group consisting of zeolite CHA, BEA, EMT, FAU (Y including ultrastabilized Y (USY), 13X (including dealuminated 13X)).

In an alternative preferred embodiment, the conversion of CO₂, optionally together with CO, involves hydrogenation to form methane. Preferably the metal is selected from the group consisting of Ni, Co, Mg, Al, Rh, Pt, Ru, Re, In, Zr, Fe, Mo, Mn and mixtures thereof, and may further comprise K, Ce, La, Na, Li, Cs, and mixtures thereof. These metals are optimally combined with a zeolite having a pore diameter in the range of 4 - 13 Å, preferably in the range of 4 - 9 Å, as sorbent. Most preferably, the sorbent in the multifunctional catalyst is a zeolite having a Si/AI ratio 1.5 - 4. The sorbent is preferably selected from the group consisting of zeolite CHA, BEA, EMT, FAU (Y including ultrastabilized Y (USY), 13X (including dealuminated 13X)).

In an alternative preferred embodiment, the conversion of CO₂, optionally together with CO, involves hydrogenation to form aromatics. In the context of this embodiment, the metal is capable of converting CO₂ into aromatic hydrocarbons. Preferably the metal is selected from the group consisting of Ga, Mo, Cu, Re, In, Zr, Au, Co, Fe, Mo, Ru and mixtures thereof, and may further comprise K, Na, Li, Cs, Mn and mixtures thereof. These metals are optimally combined with a zeolite having a pore diameter in the range of 4 - 13 Å, preferably in the range of 4 - 9 Å, as sorbent. Most preferably, the sorbent in the multifunctional catalyst is a zeolite having a Si/AI ratio above 5. The sorbent is preferably selected from the group consisting of zeolite CHA, BEA, EMT, FAU (ultrastabilized Y (USY), dealuminated X).

The multifunctional catalyst according to the invention is at least bifunctional, wherein the two functionalities are the sorption capacity and the catalytic capacity. In an especially preferred embodiment, the catalyst contains more functionalities, and preferably is trifunctional. In this embodiment, the sorbent is impregnated with a mixture of at least two metal catalysts, each of which has a different functionality.

In an especially preferred embodiment, the conversion of CO₂ involves hydrogenation to form methanol and the catalyst according to the invention contains a catalyst that is capable of catalysing the dehydration of methanol to form dimethyl ether. As such, the catalyst according to the invention is capable of efficiently converting CO₂ and H₂ into dimethyl ether in a single pass. One metal is capable of catalyzing the formation of methanol from a gaseous mixture comprising H₂ and CO₂, optionally together with CO, and another catalyst that is capable of catalysing the conversion of the thus formed methanol into dimethyl ether. The preferred catalyst for the formation of methanol is already defined above. In a preferred embodiment, the sorbent is optimized for catalyzing the dehydration of methanol. In this embodiment, the sorbent is preferably selected from faujasite (e.g.13X, zeolite Y), BEA, EMT zeolites, LTA, which are preferably subjected to dealumination and stabilization treatments as known in the art. Additionally or alternatively, the bifunctional reactive sorbent is impregnated with a further metal ("promoted"), in order to enable or promote the catalysis of dehydrating methanol to form dimethyl ether. The dehydration catalyst can be promoted by depositions of Cu, Mn, Zn and rare earth metal (e.g. Ce, La), alkali metals (Li, K), phosphor (P) or mixtures thereof, e.g. Cu-Mn-Zn supported (Zr-)HY catalyst.

In an alternative preferred embodiment, the conversion of CO₂ involves hydrogenation to form ethanol and the catalyst according to the invention further contains a catalyst that is capable of catalysing the dehydration of ethanol to form diethyl ether. As such, the catalyst according to the invention is capable of efficiently converting CO₂, optionally together with CO, and H₂ into diethyl ether in a single pass. One catalyst is capable of catalyzing the formation of ethanol from a gaseous mixture comprising H₂ and CO₂, and another catalyst is capable of converting the thus formed ethanol into diethyl ether. The preferred catalyst for the formation of ethanol is already defined above. In a preferred embodiment, the sorbent is optimized for catalyzing the dehydration of ethanol. In this embodiment, the sorbent is preferably selected from faujasite (e.g.13X, zeolite Y), BEA, EMT zeolites, LTA, which are preferably subjected to dealumination and stabilization treatments as known in the art. Additionally or alternatively, the bifunctional reactive sorbent is impregnated with a further metal ("promoted"), in order to enable or promote the catalysis of dehydrating methanol to form dimethyl ether. Further deposition of precious and transition metals can prevent formation of ethylene and boost selectivity towards diethyl ether and improve stability.

The catalyst according to the invention may also be part of a catalyst system, comprising catalytic particles according to the invention and further catalytic particles which catalyse another reaction. Especially the bifunctional catalyst as defined above may be comprised in a catalyst system, wherein a second catalytic particulate is present. For example, the further catalytic particles may be methanol dehydration catalyst particles, that catalyse the dehydration of methanol to dimethyl ether, or ethanol dehydration catalyst particles, that catalyse the dehydration of ethanol to diethyl ether. Such methanol and ethanol dehydration catalytic particles are known in the art, e.g. from Ind. Eng. Chem. Res. 1992, 31, 4, 1035-1040. Typically, these catalyst particles contain a zeolite having a Si/AI ratio above 10, such as in the range of 20 - 50. Suitable zeolites include ZSM-5; MOR, SUZ-4, FER.

As such, a gaseous feed comprising CO₂, optionally together with CO, and H₂ can be fed to a reactor comprising the catalyst system according to the invention, and be converted into dimethyl ether in a single pass.

The multifunctional catalyst according to the invention can be prepared by methods known in the art, e.g. in Catalyst Preparation: Science and Engineering, Regalbuto (2007), CRC Press (ISBN 978-0-8493-7088-5), or in US3226339 and US3527836. In a preferred embodiment, the catalyst according to the invention is prepared according to the following process:
(i) contacting a solid water sorbent with a solution comprising at least one metal capable of catalysing the synthesis of CO from a gaseous mixture comprising H₂ and CO₂ via RWGS;
(ii) drying at a temperature in the range of 90 - 150 °C, preferably 95 - 115 °C;
(iii) calcinating at a temperature in the range of 200 - 500 °C, preferably 250 - 450 °C, more preferably 300 - 400 °C;
(iv) activating the catalyst under reducing atmosphere.

The sorbent subjected to step may is typically in powder form. Preferably, the sorbent powder has an average particle size in the range of 50 - 200 µm, more preferably in the range of 100 - 150 µm.

In step (i), the sorbent is impregnated with the metal solution. The volume of the solution is normally 0.5 to 10 times, preferably 1 to 5 times, the pore volume of sorbent. The pore volume of the material may be determined by N₂ adsorption-desorption type of experiments or by a parallel wetting experiment to determine the level of dryness achieved by the event of capillary condensation as a function of the volume added. In a preferred embodiment, the volume of the solution is 1 to 4 times, more preferably 1.5 to 3.5 times, most preferably 2 to 3 times, the pore volume of sorbent. The metal is typically dissolved in water in salt form, such as Pt(NH₃)₄(NO₃)₂. This step is typically performed within a few minutes at room temperature and atmospheric pressure. The volume of the solution and the concentration of the metal therein should be chosen such that the final catalyst, obtained in step (iii), contains metal in an amount of at most 40 wt% based on total weight of the catalyst.

In step (ii), the solvent is removed, to obtain a dried layer of metal precursor on the surface of the sorbent. Drying is typically performed at a temperature in the range of 90 - 150 °C in an oven, preferably in the range of 95 - 105 °C. This step is usually performed at atmospheric pressure under air atmosphere.

In step (iii), the metal is converted into its oxide by calcination, which ensures dispersion of the metal over the surface of the sorbent. Any means for calcination known in the art may be sued. Calcination is typically performed at a temperature in the range of 200 - 500 °C, preferably in the range of 250 - 450 °C, more preferably in the range of 300 - 400 °C. This step is usually performed in an oven under air atmosphere with a ramp rate of 5 °C/min to the designated temperature, the temperature that was allowed to reduce after 6 hours to room temperature.

In step (iv), the metal precursor is converted into the catalytically active form of the metal. The skilled person is familiar with activation of metal catalysts. Activation is performed under a reducing atmosphere, typically an atmosphere comprising hydrogen gas. The samples are typically heated in-situ to 180 °C at 5 °C/min under 10% hydrogen in N₂, after which the heating rate was reduced to 2 °C/min up to 325 °C followed by a 30 minute temperature hold and subsequently cooled down to reaction temperature.

During step (iii), the catalyst is activated, and is typically performed as last step shortly prior to the RWGS reaction is carried out. Normally, the activation is performed within the RWGS reactor as first step of the RWGS process. Does, the process may further comprise a step wherein the dried catalyst of step (ii) is loaded into a RWGS reactor, wherein step (iii) is performed.

If needed, the dried catalyst of step (ii) may be converted into a granulate, before it is subjected to the RWGS reactor. This conversion may be performed by compressing the dried catalyst and broken into granules, e.g. by milling. The compression step may be performed using a hydraulic press and a compression force of for example 2 - 6 atm.

This process, comprising steps (i) - (iv), is particularly advantageous, because it enables the impregnation of the solid water sorbent with a metal catalyst without significant reduction of its water sorption capacity. In one aspect, the invention also concerns the process for preparing the catalyst as defined herein.

### Process

The invention further concerns a process for the conversion of CO₂ from a gaseous feedstock comprising H₂ and CO₂, comprising the step of subjecting the gaseous feedstock to the multifunctional catalyst according to the invention. The process may also be referred as being for the formation of a species selected from CO, methane, methanol, ethanol, dimethyl ether, diethyl ether, alkanes, alkenes or aromatics. The conversion of CO₂ preferably involves a reverse water gas shift reaction (RWGS) to form CO, a hydrogenation reaction to form methanol or ethanol, a Fisher-Tropsch reaction to form alkanes, a Fisher-Tropsch reaction to form alkenes, a Methanol to Olefins (MTO) reaction to form alkenes, an aromatization reaction to form aromatic hydrocarbons or a hydrogenation reaction to form methane, optionally wherein the hydrogenation reaction is accompanied with a dehydration reaction to convert methanol into dimethyl ether or ethanol into diethyl ether. In an especially preferred embodiment, the process is for the formation of CO via the RWGS reaction.

The conversion of CO₂ using catalytic particles is known in the art. The contacting between the gaseous feedstock with the catalyst is typically performed in a fixed bed or fluidized bed reactor. The skilled person is capable of determining the optimal conditions for the desired conversion, depending on the specific desired product. In general, the process is typically performed at a temperature in the range of 100 - 500 °C, preferably in the range of 200 - 400 °C. Likewise, it is preferred that the process takes place at a pressure in the range of 0.5 - 20 MPa, preferably 1 - 10 MPa, more preferably 1.5 - 5 MPa.

In one embodiment, the process according to the invention is a RWGS reaction, and is thus performed under conditions suitable for conducting a RWGS reaction. In a preferred embodiment, the RWGS reaction takes place at a temperature in the range of 150 - 350 °C, preferably in the range of 290 - 330 °C. Likewise, it is preferred that the RWGS reaction takes place at a pressure in the range of 0.1 - 5.0 MPa, preferably 0.5 - 4.0 MPa, more preferably 1 - 2.5 MPa. These conditions have were found to be ideally suitable for performing the RWGS reaction with the catalyst according to the present invention. A preferred catalyst for the RWGS reaction is defined above, and is preferably used in the process according to the present embodiment.

In an alternative embodiment, the process according to the invention is a hydrogenation reaction to convert CO₂ into methanol, and is performed under conditions suitable for conducting such a hydrogenation reaction. In a preferred embodiment, the reaction takes place at a temperature of 200 - 400 °C, more preferably 230 - 320 °C, most preferably at about 300 °C. A preferred catalyst for the hydrogenation reaction is defined above, and is preferably used in the process according to the present embodiment.

In an alternative embodiment, the process according to the invention is a hydrogenation reaction to convert CO₂ into ethanol, and is performed under conditions suitable for conducting such a hydrogenation reaction. In a preferred embodiment, the reaction takes place at a temperature of 200 - 400 °C, more preferably 230 - 320 °C, most preferably at about 300 °C. A preferred catalyst for the hydrogenation reaction is defined above, and is preferably used in the process according to the present embodiment.

In an alternative embodiment, the process according to the invention is a Fischer-Tropsch reaction to convert CO₂ into alkanes, and is performed under conditions suitable for conducting such a Fischer-Tropsch reaction. In a preferred embodiment, the reaction takes place at a temperature of 200 - 400 °C, more preferably 250 - 350 °C, most preferably at about 320 °C. A preferred catalyst for the Fischer-Tropsch reaction for preparing alkanes is defined above, and is preferably used in the process according to the present embodiment.

In an alternative embodiment, the process according to the invention is a Fischer-Tropsch reaction to convert CO₂ into alkenes, and is performed under conditions suitable for conducting such a Fischer-Tropsch reaction. In a preferred embodiment, the reaction takes place at a temperature of 200 - 400 °C, more preferably 250 - 350 °C, most preferably at about 320 °C. A preferred catalyst for the Fischer-Tropsch reaction for preparing alkenes is defined above, and is preferably used in the process according to the present embodiment.

In an alternative embodiment, the process according to the invention is a Methanol to Olefin reaction to convert CO₂ into alkenes, and is performed under conditions suitable for conducting such a MTO reaction. In a preferred embodiment, the reaction takes place at a temperature of 200 - 400 °C, more preferably 250 - 350 °C, most preferably at about 320 °C. A preferred catalyst for the MTO reaction is defined above, and is preferably used in the process according to the present embodiment.

In an alternative embodiment, the process according to the invention is a methanation reaction to convert CO₂ into methane, and is performed under conditions suitable for conducting such a methanation reaction. In a preferred embodiment, the reaction takes place at a temperature of 200 - 400 °C, more preferably 250 - 350 °C, most preferably at about 320 °C. A preferred catalyst for the methanation reaction is defined above, and is preferably used in the process according to the present embodiment.

In an alternative embodiment, the process according to the invention is an aromatization reaction to convert CO₂ into aromatic hydrocarbons, and is performed under conditions suitable for conducting such an aromatization reaction. In a preferred embodiment, the reaction takes place at a temperature of 200 - 400 °C, more preferably 250 - 350 °C, most preferably at about 320 °C. A preferred catalyst for the aromatization reaction is defined above, and is preferably used in the process according to the present embodiment.

In an alternative embodiment, the process according to the invention is a hydrogenation reaction to convert CO₂ into methanol and a dehydration reaction to convert methanol into dimethyl ether, and is performed under conditions suitable for conducting such a hydrogenation and dehydration reaction. In a preferred embodiment, the reaction takes place at a temperature of 200 - 400 °C, more preferably 225 - 300 °C, most preferably at about 275 °C. Preferred catalysts for the combined hydrogenation and dehydration reaction is defined above, and is preferably used in the process according to the present embodiment. Herein, both the catalyst system comprising two distinct catalytic particles, one of which is the bifunctional catalyst according to the invention, and the trifunctional catalyst according to the invention is suitable.

In an alternative embodiment, the process according to the invention is a hydrogenation reaction to convert CO₂ into ethanol and a dehydration reaction to convert ethanol into diethyl ether, and is performed under conditions suitable for conducting such a hydrogenation and dehydration reaction. In a preferred embodiment, the reaction takes place at a temperature of 200 - 400 °C, more preferably 225 - 300 °C, most preferably at about 275 °C. A preferred catalyst for the combined hydrogenation and dehydration reaction is defined above, and is preferably used in the process according to the present embodiment. Herein, both the catalyst system comprising two distinct catalytic particles, one of which is the bifunctional catalyst according to the invention, and the trifunctional catalyst according to the invention is suitable.

The feedstock of the process according to the present invention is a gaseous feedstock comprising H₂ and CO₂. The feedstock may contain further gaseous components, such as CO, gaseous contaminants of a process gas (e.g. H₂S, CH4) and inert gases (e.g. N₂, Ar). The conversion of CO₂ in CO and possibly downstream products is an important aspect of the present invention. For the process according to the invention, the presence of some CO in the feedstock has no negative impact. Also, the process according to the invention is capable of dealing with minor contaminants of CO₂-containing process streams. In case the invention concerns the RWGS reaction, it is preferred that the feedstock does not comprise CO, as CO is efficiently formed in the RWGS reaction according to the present invention.

Preferably, the feedstock contains H₂ and CO₂, and optionally CO and inert gases such as N₂ and Ar, and no other gaseous species. The molar ratio of H₂ to CO₂ in the feedstock may be in the range of 25:1 - 1:10, preferably 10:1 - 1:5, more preferably 5:1 - 1:1. The skilled person is capable of optimizing the molar ratio of in the feedstock for the desired product. For example, the optimal H₂ to CO₂ ratio for the RWGS reaction is about 1:1, while for alcohol, ether or alkanes, the feedstock preferably contains an excess of H₂, such as an H₂ to CO₂ ratio of about 2:1 - 4:1.

The process according to the invention preferably comprises a step wherein the catalyst is regenerated. During the reaction, water molecules are sorbed onto the sorbent, in order to shift the equilibrium of the reaction towards the product, such as CO in case of the RWGS reaction. During regeneration, the sorbed water molecules are desorbed from the sorbent, such that the sorbent becomes available for a further reaction. Regeneration may be accomplished by any method known in the art. Typically, a temperature swing and/or pressure swing adsorption is employed. In one embodiment, the regeneration step involves a pressure reduction by at least 0.1 x 10⁶ Pa, preferably to a pressure in the range of 0.1 x 10⁶ Pa - 0.3 x 10⁶ Pa, and/or a temperature increase by at least 20 °C, preferably to a temperature in the range of 370 °C - 500 °C.

### Use

A third aspect of the invention relates to the use of the catalyst according to the invention as catalyst for the RWGS reaction. The use according to the present aspect may also be worded as the use of the catalyst according to the invention as catalyst for the synthesis of CO via the RWGS reaction. The use according to the present aspect preferably employs the process according to the invention, and everything defined for the process according to the invention equally applies to the use according to the invention.

### EXAMPLES

The invention is further illustrated by the following non-limiting examples.

### Example 1: Preparation of multifunctional catalysts

A range of multifunctional catalysts according to the invention (1) - (6) was prepared. Selected solid water sorbents were contacted with a solution comprising a catalytic metal (see Table below for details per catalyst). The catalyst was dried at 105 °C under rigorous stirring. Subsequently, the catalysts were calcined at 400 °C (5 °C/min, 6h) to remove volatile components, depositing the metal oxide clusters on the catalyst surface. Next, the granulate was compressed using a pill press at 4 atm, then broken into granules. A sieve fraction of 212-450 µm was selected for the experiment. The last step in the preparation of the catalyst preparation is reduction in hydrogen flow (typically this step is carried out in-situ, the first step the catalysts in the reactor is subjected to).

Besides the multifunctional catalysts, a conventional catalyst typically used in RWGS reactions was prepared for comparison purposes.

| | **Sorbent** | **Metal** | **Impregnation procedure** |
|---|---|---|---|
| (**1**) | Zeolite 13X | 10 wt% Cu | >106 µm powder impregnated with 0.407 g Cu(NO₃)₂•2.5H₂O per gram of zeolite material by dissolving in the minimal amount of water. |
| (**2**) | Zeolite LTA-4A | 10 wt% Cu 7 wt% Zn | >106 µm powder impregnated with 0.407 g Cu(NO₃)₂•2.5H₂O and 0.176 g ZnCl₂ per gram of zeolite material by dissolving in the minimal amount of water. |
| (**3**) | Zeolite 13X | 7 wt% Cu 3 wt% Zn | >106 µm powder impregnated with 0.285 g Cu(NO₃)₂•2.5H₂O and 0.123 g Zn(NO3)2•3H2O per gram of zeolite material by dissolving in the minimal amount of water. |
| (**4**) | Zeolite 13X | 2 wt% Pt | >106 µm powder impregnated with 0.041 g Pt(NH₃)₄(NO₃)₂ per gram of zeolite material by dissolving in the minimal amount of water. |
| (**5**) | Zeolite LTA-4A | 2 wt% Pt | >106 µm powder impregnated with 0.041 g Pt(NH₃)₄(NO₃)₂ per gram of zeolite material by dissolving in the minimal amount of water. |
| (**6**) | Zeolite LTA-5A | 2 wt% Pt | >106 µm powder impregnated with 0.041 g Pt(NH₃)₄(NO₃)₂ per gram of zeolite material by dissolving in the minimal amount of water. |

### Example 2: CO selectivity during RWGS

Small-scale RWGS processes were conducted four of the catalysts of example 1, as well as with control catalyst (7), a low- temperature CuZnAI commercial water gas shift mixed (LTS) with molsieves 3A sorbent (ms). A feedstock of H₂ and CO₂ (4/1 mol/mol) and 5 % of argon as tracer was fed to 26 g of the catalyst at 300 °C and 10 bar(a). Regeneration of the catalyst was performed by a combined temperature and pressure swing (TPSA), by lowering the pressure to 5 bar(a) and heating to 400 °C, while feeding N₂. The composition of the product gas was analysed by gas chromatography and mass spectroscopy. The composition (in mol%) is given in the table below.

| | **CH₄** | **CO** | **CO₂** | **C₂H₄** | **DME** | **CH₃OH** |
|---|---|---|---|---|---|---|
| (**1**) | 1.9 | 73.3 | 23.4 | 0.9 | 0.6 | 0 |
| (**4**) | 13 | 72.3 | 13.7 | 0.2 | 0.4 | 0.4 |
| (**5**) | 5.9 | 85.1 | 7.8 | 0.9 | 0.2 | 0.1 |
| (**6**) | 69.2 | 1.5 | 29 | 0.3 | 0 | 0 |
| (**7**) | 4.6 | 57.2 | 15.8 | 0.7 | 21.2 | 0.5 |

All inventive catalysts outperformed the conventional catalyst (7), which is not selective for CO and forms substantial amounts of DME. Catalyst (5), a LTA-4A sorbent impregnated with 2 wt% Pt, gave especially good results, with over 90 % conversion to CO and hardly any DME and CH₄ formed.

### Example 3: Stability

An extended RWGS process (> 71 days) was conducted with catalyst (5) of example 1. A feedstock of H₂ and CO₂ (4/1 mol/mol) and 5 % of argon as tracer was fed to 26 g of the catalyst at 300 °C and 10 bar(a). Regeneration of the catalyst was performed as part of each cycle after each reactive adsorption period of 2.5 hours by a combined temperature and pressure swing (TPSA), by lowering the pressure to 3 bar(a) and heating to 400 °C, while feeding N₂. The composition of the product gas was analysed periodically by gas chromatography and mass spectroscopy. The composition (in mol%) at certain points in time is given in the table below.

| **Time (h)** | **S_max (CO)** | **S_max (CO₂)** | **S_max (DME)** | **S_max (MeOH)** | **S_max (CH₄)** |
|---|---|---|---|---|---|
| 432 | 85.7 | 7.9 | 0.3 | 0.2 | 6.0 |
| 672 | 82.5 | 13.1 | 0.0 | 0.0 | 4.4 |
| 1152 | 92.2 | 2.8 | 0.0 | 0.0 | 5.0 |
| 1704 | 97.8 | 2.2 | 0.0 | 0.0 | 0.0 |

Surprisingly, the catalyst was activated over time, while a (slight) deactivation normally occurs with conventional catalysts during such extended experiments. After 71 days, the catalyst according to the invention gave excellent conversions towards CO.

### Example 4: Tuning the design of catalyst toward selective formation of dimethyl ether

Zeolite 13X based catalysts **(1)** and **(4)** of Example 1 were compared using a feedstock of H₂:CO:CO₂ = 8:1:2 at 25 bar and 275 °C. The Cu based catalyst **(1)** showed approximately 10 times higher dimethyl ether yield compared to the Pt analogue **(4).**

## Claims

1. Use of a multifunctional catalyst, comprising a solid water sorbent impregnated with at least one metal capable of converting CO₂ from a gaseous mixture comprising H₂ and CO₂, as catalyst for the conversion of CO₂, wherein the sorbent is a molecular sieve having a Si/AI molar ratio below 50 and containing pores having a diameter in the range of 3 to 20 Å and the metal content in the catalyst is not more than 40% based on of total weight of the multifunctional catalyst.

2. The use according to claim 1, wherein the conversion of CO₂ involves a reverse water gas shift reaction (RWGS) to form CO, a hydrogenation reaction to form methanol or ethanol, or a Fisher-Tropsch reaction to form alkanes, a Fisher-Tropsch reaction to form alkenes, a Methanol to Olefins (MTO) reaction to form alkenes, an aromatization reaction to form aromatic hydrocarbons or a hydrogenation reaction to form methane, optionally wherein the hydrogenation reaction is accompanied with a dehydration reaction to convert methanol into dimethyl ether or ethanol into diethyl ether.

3. A process for the conversion of CO₂ from a gaseous feedstock comprising H₂ and CO₂, comprising the step of subjecting the gaseous feedstock to a multifunctional catalyst, comprising a solid water sorbent impregnated with at least one metal capable of converting CO₂ from a gaseous mixture comprising H₂ and CO₂, wherein the sorbent is a molecular sieve having a Si/AI molar ratio below 50 and containing pores having a diameter in the range of 3 to 20 Å and the metal content in the catalyst is not more than 40% based on total weight of the multifunctional catalyst.

4. The process according to claim 3, wherein the conversion of CO₂ involves a reverse water gas shift reaction (RWGS) to form CO, a hydrogenation reaction to form methanol or ethanol, or a Fisher-Tropsch reaction to form alkanes, a Fisher-Tropsch reaction to form alkenes, a Methanol to Olefins (MTO) reaction to form alkenes, an aromatization reaction to form aromatic hydrocarbons or a hydrogenation reaction to form methane, optionally wherein the hydrogenation reaction is accompanied with a dehydration reaction to convert methanol into dimethyl ether or ethanol into diethyl ether.

5. The process according to claim 4, wherein the conversion is a RWGS reaction.

6. The process according to any one of claims 3 - 5, wherein the process takes place at a temperature in the range of 100 - 500 °C, preferably in the range of 200 - 400 °C.

7. The process according to any one of claims 3 - 6, wherein the reaction takes place at a pressure in the range of 0.5 - 20 MPa, preferably 1 - 10 MPa, more preferably 1.5 - 5 MPa.

8. The process according to any one of claims 3 - 7, wherein the molar ratio of H₂ to CO₂ in the feedstock is in the range of 10:1 - 1:5, preferably 5:1 - 1:1, more preferably about 4:1, and/or wherein the feedstock further comprises CO.

9. The process according to any one of claims 3 - 8, further comprising a regeneration step of the catalyst wherein the pressure is reduced by at least 0.1 x 10⁶ Pa, preferably to a pressure in the range of 0.1 x 10⁶ Pa - 0.3 x 10⁶ Pa and/or the temperature is increased by at least 20 °C, preferably to a temperature in the range of 370 °C - 500 °C.

10. A multifunctional catalyst, comprising a solid water sorbent impregnated with at least one metal capable of catalyzing the conversion of CO₂ from a gaseous mixture comprising H₂ and CO₂, wherein the sorbent is a molecular sieve having a Si/AI molar ratio below 50 and containing pores having a diameter in the range of 3 to 20 Å and the metal content in the catalyst is not more than 40% based on total weight of the multifunctional catalyst.

11. The use according to claim 1 or 2, the process according to any one of claims 3 - 9, or the catalyst according to claim 10, wherein the sorbent has a Si/AI molar ratio in the range of 1 and 2, more preferably in the range of 1 and 1.5, most preferably the Si/AI molar ratio is 1, preferably wherein the sorbent is a zeolite selected from LTA zeolite, X-type zeolite and mixtures thereof, preferably the multifunctional component comprises LTA-4A zeolite and/or zeolite 13X.

12. The use according to claim 1, 2 or 11, the process according to any one of claims 3 - 9 and 11, or the catalyst according to claim 10 or 11, wherein the pores have a diameter in the range of 4 to 8 Å.

13. The use according to claim 1, 2, 11 or 12, the process according to any one of claims 3 - 9, 11 and 12, or the catalyst according to any one of claims 10 -12, wherein the internal and external surfaces of the sorbent are impregnated with the at least one metal catalyst, and/or wherein the sorbent is impregnated with 0.1 - 40 wt%, preferably 1 - 30 wt%, of the metal, based on total weight of the multifunctional catalyst.

14. The use according to any one of claims 1, 2, 11 - 13, the process according to any one of claims 3 - 9, 11 - 13, or the catalyst according to any one of claims 10 -13, wherein the at least one metal is selected from the group consisting of Pt, Cs, Cu, Rh, Au, Zn, Co, Fe, Mo, Zn, Mn, Ru, Ir, Os, Ag, Al, Cr, Li, Na, K, Rb, Cs, Ce, Re, In, Zr, Ni, Mg, Al, Pd, Ga and mixtures thereof, preferably from Pt, Cu, Rh, Ir, Ru, Pd, Os, Cs, Fe, Zn and mixtures thereof, more preferably from the group consisting of Pt, Zn, Fe, Cs, Cu and mixtures thereof.

15. The use according to any one of claims 1, 2, 11 - 14, the process according to any one of claims 3 - 9, 11 - 14, or the catalyst according to any one of claims 10 - 14, wherein the solid sorbent is further impregnated with a second metal capable of catalyzing the synthesis of dimethyl ether from methanol or diethyl ether from ethanol.
